Europäisches Patentamt

European Patent Office

Office européen des brevets

(19)

(11) **EP 0 558 996 B1**

(12) **EUROPÄISCHE PATENTSCHRIFT**

(45) Veröffentlichungstag und Bekanntmachung des Hinweises auf die Patenterteilung:
**01.05.1996  Patentblatt 1996/18**

(51) Int. Cl.$^6$: **C07C 68/00**, C07C 69/96

(21) Anmeldenummer: **93102461.6**

(22) Anmeldetag: **17.02.1993**

(54) **Verfahren zur Herstellung von Dialkylcarbonaten**

Process for the preparation of dialkyle carbonates

Procédé de préparation de carbonates de dialkyle

(84) Benannte Vertragsstaaten:
**BE CH DE FR GB IT LI NL**

(30) Priorität: **02.03.1992 DE 4206527**

(43) Veröffentlichungstag der Anmeldung:
**08.09.1993  Patentblatt 1993/36**

(73) Patentinhaber: **BAYER AG**
**D-51368 Leverkusen (DE)**

(72) Erfinder:
- **Wolters, Erich, Dr.**
  **W-5000 Köln 41 (DE)**
- **Landscheidt, Heinz, Dr.**
  **W-4100 Duisburg 1 (DE)**
- **Klausener, Alexander, Dr.**
  **W-5190 Stolberg (DE)**
- **Puppe, Lothar, Dr.**
  **W-5093 Burscheid (DE)**

(56) Entgegenhaltungen:
**EP-A- 0 464 460          EP-A- 0 523 508**

**Beschreibung**

Die vorliegende Erfindung betrifft ein Verfahren zur Herstellung von Dialkylcarbonaten durch Umsetzung von Kohlenmonoxid (CO) mit Alkylnitriten in Gegenwart eines Katalysators aus der Reihe der Platinmetall-Halogenide auf einem Träger aus amorphen Alumosilikat, der Zusätze von Verbindungen weiterer Elemente enthalten kann.

Dialkylcarbonate sind von allgemeiner chemischer Bedeutung. So ist beispielsweise Diethylcarbonat ein ausgezeichnetes Lösungsmittel im mittleren Siedebereich. Des weiteren sind die Dialkylcarbonate ausgezeichnete Carbonylierungs- und Acylierungsreagenzien. Schließlich haben sie große Bedeutung bei der Herstellung von anderen Carbonaten, von Urethanen und von Harnstoffen.

Es ist bekannt, Dialkylcarbonate durch Umsetzungen von Phosgen bzw. von Chlorameisensäurealkylestern mit Alkoholen herzustellen. Es besteht jedoch ein steigendes Interesse daran, den Einsatz des giftigen Phosgens bzw. der davon abgeleiteten Zwischenprodukte, wie der Chlorameisensäureester, durch andere Verfahren abzulösen. Neben Versuchen, Dialkylcarbonate durch Umsetzung von CO mit niederen Alkoholen zu erhalten, sind insbesondere Verfahren wichtig, in denen CO in der Gasphase mit Alkylnitrit an einem Platinmetall-Katalysator umgesetzt werden. Bei solchen Umsetzungen wird neben dem gewünschten Dialkylcarbonat stets das Auftreten von Dialkyloxalat beobachtet.

So ist aus EP 425 197 ein Verfahren bekannt, das gemäß seiner bevorzugten Ausführungsform zu Dialkylcarbonaten des Methanols bzw. Ethanols aus CO und Methyl- bzw. Ethylnitrit in der Gasphase an einem $PdCl_2$-Katalysator auf Aktivkohle führt. Die Selektivitäten zu den gewünschten niederen Dialkylcarbonaten erreichen gemäß dieser EP 425 197, Tabelle 1, Werte bis zu 94 %; jedoch werden als Nebenprodukte stets niedrige Dialkyloxalate und $CO_2$ beobachtet. Bei einer Nacharbeitung konnten darüber hinaus die angegebenen hohen Selektivitäten nur ungenügend reproduziert werden. Die Katalysatoren dieser EP 425 197 enthalten Zusätze von Chloriden unedler Metalle; ein beträchtlicher Zusatz von Chlorwasserstoff, nämlich eine Menge von 1 bis 50 mol-%, bezogen auf das Platinmetall im Katalysator, wird dem System zugesetzt oder ein Teil des Katalysators muß aus dem Reaktor entnommen werden und einer Behandlung mit Chlorwasserstoff unterzogen werden.

Auch in der Zeitschrift für Katalytische Forschung (China), Vol. 10 (1), S. 75-78 (1989), wird als Träger für einen $PdCl_2$-haltigen Katalysator ein Träger aus Kohle eingesetzt, um aus CO und Methylnitrit Dimethylcarbonat zu erhalten, wobei jedoch stets nebenher zusätzlich das Dimethyloxalat entsteht.

Ein Pd/Kohle-Katalysator wird auch in Chin. Sci. Bull. <u>34</u> (1989), 875-76 für die Herstellung von Dimethylcarbonat aus CO und Methylnitrit erwähnt.

Diese Bevorzugung eines Kohleträgers ist nicht unerwartet, da in Platinum Metals Review <u>34</u> (1990), 178-180 unter Bezugnahme auf ältere Literatur davon berichtet wird, daß bei der Umsetzung eines niederen Alkylnitrits mit CO an einem Pd-Katalysator je nach dem Träger verschiedene Hauptprodukte erhalten werden; ein Kohle-Träger ergibt demnach überwiegend die Dialkylcarbonate, während ein oxidischer Träger wie z.B. ein $Al_2O_3$-Träger in der Hauptsache Dialkyloxalate ergibt.

Es wurde nun gefunden, daß amorphe Alumosilikate als Katalysatorträger nicht nur in unerwarteter Weise zu den Dialkylcarbonaten führt, wenn man CO mit Alkylnitriten umsetzt, sondern es wurde darüber hinaus auch noch eine bedeutende Steigerung der Selektivität zu den gewünschten Dialkylcarbonaten erzielt, die soweit geht, daß neben mehr als 97 % Selektivität, in vielen Fällen mehr als 99 % Selektivität zu diesen Dialkylcarbonaten im allgemeinen überhaupt kein Oxalat nachgewiesen werden kann. Lediglich eine geringe Menge an $CO_2$ kann im Reaktionsgemisch beobachtet werden. Darüber hinaus hat die für nicht möglich gehaltene Verwendung der amorphen Alumosilikate als Träger den wesentlichen Vorteil einer erhöhten Stabilität und Abriebfestigkeit, verglichen mit einem Trägerkatalysator auf der Basis eines Kohleträgers.

Es wurde ein Verfahren zur Herstellung von Dialkylcarbonaten der Formel

$$O=C(OR)_2 \qquad (I),$$

worin

R    für geradkettiges oder verzweigtes $C_1$-$C_4$-Alkyl steht,

durch Umsetzung von Kohlenmonoxid (CO) mit Alkylnitriten der Formel

$$RONO \qquad (II),$$

worin

R    die angegebene Bedeutung hat,

in Anwesenheit oder Abwesenheit eines Inertgases sowie in Anwesenheit oder Abwesenheit des zugrundeliegenden Alkohols ROH sowie in Anwesenheit oder Abwesenheit von NO an einem Platinmetall-Träger-Katalysator bei erhöhter Temperatur in kontinuierlicher Gasphasenreaktion gefunden, das dadurch gekennzeichnet ist, daß als Träger amorphe Alumosilikate mit einem Gehalt von 30 - 80 Atom-% Al, bezogen auf die Summe von Al und Si, eingesetzt werden, das Platinmetall in Form eines Halogenids oder einer Halogenid enthaltenden Komplexverbindung vorliegt, wobei das Platinmetallhalogenid oder der das Platinmetall enthaltende halogenhaltige Komplex in situ im Verfahrensreaktor aus dem Platinmetall oder einer halogenfreien Platinmetallverbindung mit Hilfe von Halogenwasserstoff unter den Reaktionsbedingungen gebildet werden kann, der Katalysator weiterhin mit oder ohne einen Zusatz aus einer Verbindung von Antimon, Wismut, Aluminium, Kupfer, Vanadium, Niob, Tantal, Zinn, Eisen, Kobalt, Nickel oder einem Gemisch mehrerer von ihnen ausgerüstet ist und bei einem Volumenverhältnis von Nitrit:CO = 0,1-10:1 und einer Temperatur von 50-150°C gearbeitet wird, wobei absatzweise oder kontinuierlich Halogenwasserstoff mindestens in der Menge nachgesetzt wird, die auch ohne Halogenwasserstoffzusatz mit dem Reaktionsgemisch aus dem Reaktor herausgetragen wird.

Amorphe Alumosilikate als erfindungsgemäß einzusetzende Träger sind Gemenge (Gemische) von $Al_2O_3$ und $SiO_2$ in stark wechselnden Verhältnissen, die teilweise Verbindungscharakter haben. Der Anteil an Al beträgt 30 - 80 Atom-%, bevorzugt 50 - 75 Atom-%, bezogen auf die Summe von Al und Si. Es können Anteile von (Erd)Alkalimetalloxiden vorliegen, etwa im Bereich von 0,01 - 30 Gew.-%, bezogen auf das Gesamtgewicht des amorphen Alumosilikats. Sie haben für den erfindungsgemäßen Einsatz BET-Oberflächen von 100-1 000 $m^2/g$, bevorzugt 200-800 $m^2/g$.

Solche Alumosilikate finden sich als natürliche Rohstoffe, können aber auch synthetisch durch gemeinsames Fällen von $Al_2O_3$ und $SiO_2$ aus Aluminat- und Silikatlösungen hergestellt werden. Ein typischer Vertreter ist $Al_2O_3 \cdot SiO_2$. Durch Trocknung und Kalzinieren kann die Oberfläche von amorphen Alumosilikaten in einer dem Fachmann geläufigen Weise variiert werden. Bei höheren spezifischen Oberflächen gewinnen die amorphen Alumosilikaten in zunehmendem Maße saure Eigenschaften. Solche Alumosilikate mit sauren Eigenschaften sind erfindungsgemäß bevorzugt.

Die Reaktion im erfindungsgemäßen Verfahren erfolgt im Sinne der nachfolgenden Reaktionsgleichung:

$$CO + 2\ RONO \rightarrow O{:}C(OR)_2 + 2\ NO.$$

Geradkettiges oder verzweigtes Alkyl mit 1-4 C-Atomen ist beispielsweise Methyl, Ethyl, Propyl, Isopropyl, Butyl, Isobutyl, in bevorzugter Weise die genannten n-Alkyle, in besonders bevorzugter Weise Methyl und Ethyl und in ganz besonders bevorzugter Weise Methyl.

Grundsätzlich ist es möglich, von einem Gemisch verschiedener Alkylnitrite auszugehen, wobei jedoch auch ein Gemisch verschiedener Dialkylcarbonate und gegebenenfalls unsymmetrisch substituierte Dialkylcarbonate entstehen. Im Sinne einer einheitlichen Reaktion ist es daher bevorzugt, von nur einem Alkylnitrit auszugehen.

Während es grundsätzlich möglich ist, CO mit einem Alkylnitrit ohne weitere Gemischkomponente umzusetzen, beispielsweise wenn man sich in der Gemischzusammensetzung außerhalb der Explosionsgrenzen befindet, wird vielfach ein Inertgas zur Verdünnung der Reaktionspartner herangezogen. Inertgase sind beispielsweise Edelgase, Stickstoff und Kohlendioxid, bevorzugt Argon, Stickstoff oder Kohlendioxid, besonders bevorzugt Stickstoff und Kohlendioxid. Die Menge des Inertgases beträgt 20-80 Vol.-%, bevorzugt 30-70 Vol.-%, bezogen auf das gesamte in den Reaktor einzuführende Gasvolumen. Das Inertgas und gegebenenfalls darin enthaltene nicht umgesetzte Restmengen der Reaktionspartner können recyclisiert werden.

Das Volumenverhältnis der Reaktionspartner Nitrit und CO unter sich beträgt 0,1-10:1, bevorzugt 0,2-4:1, besonders bevorzugt 0,3-3:1.

Das umzusetzende Gasgemisch kann weiterhin geringe Mengen an Alkohol ROH, beispielsweise in einer Menge von 0-10 Vol.-% und geringe Mengen an NO, beispielsweise in einer Menge von 0-10 Vol.-%, beides bezogen auf das Gesamtvolumen des einzusetzenden Gasgemisches, enthalten. Solche Zusätze an ROH bzw. NO können etwa aus der Herstellung des Alkylnitrits stammen und beispielsweise mit diesem in das Reaktionsgasgemisch hereingebracht werden.

Der Katalysator für das erfindungsgemäße Verfahren wird auf die amorphen Alumosilikate als Träger aufgebracht; seine Reaktivkomponente besteht im reaktionsbereiten Zustand aus dem Platinmetall-Halogenid oder der Platinmetall-Halogenid enthaltenden Komplexverbindung. Solche Komplexverbindungen sind grundsätzlich bekannt und sind beispielsweise Alkalimetallchlorid-Komplexverbindungen, wie Lithium- oder Natrium-tetrachloropalladat, $Li_2[PdCl_4]$ bzw. $Na_2[PdCl_4]$.

Es hat sich weiterhin gezeigt, daß das Platinmetall-Halogenid bzw. das Platinmetall-Halogenid enthaltende Komplexverbindung in situ im Reaktor aus metallischem Platinmetall oder einer halogenfreien Platinmetall-Verbindung unter den Reaktionsbedingungen, d.h. in Gegenwart des umzusetzenden Gasgemisches, mit Hilfe von Halogenwasserstoff gebildet werden kann. Der Reaktor kann demnach auch mit einem ansonsten vergleichbaren Katalysator gefüllt werden, der das Platinmetall zunächst in metallischer Form enthält oder der mit Hilfe einer halogenfreien Platinmetall-Verbindung hergestellt worden war. Solche halogenfreien Platinmetall-Verbindungen, die hierfür in Frage kommen, sind beispielsweise Platinmetall-nitrate, -propionate, -butyrate, -carbonate, -oxide, -hydroxide oder andere dem Fachmann geläufige.

Elemente der Gruppe der Platinmetalle im Sinne der Erfindung sind Palladium, Platin, Iridium, Ruthenium und Rhodium, bevorzugt Palladium, Ruthenium und Rhodium, besonders bevorzugt Palladium.

Halogenide im Sinne der Erfindung sind Fluorid, Chlorid, Bromid und Iodid, bevorzugt Chlorid und Bromid, besonders bevorzugt Chlorid.

Die Menge des Platinmetall-Halogenids oder der das Platinmetall-Halogenid enthaltenden Komplexverbindung beträgt 0,01-8 Gew.-%, bevorzugt 0,05-4 Gew.-%, gerechnet als Platinmetall und bezogen auf das Gesamtgewicht des Katalysators.

Der Katalysator für das erfindungsgemäße Verfahren ist weiterhin mit oder ohne einen Zusatz aus einer Verbindung von Antimon, Wismut, Aluminium, Kupfer, Vanadium, Niob, Tantal, Zinn, Eisen, Kobalt, Nickel oder einem Gemisch mehrerer von ihnen ausgerüstet; in einer bevorzugten Weise liegt ein solcher Zusatz vor.

Solche Zusätze liegen in salzartiger oder in metallischer Form der genannten Elemente vor. In ähnlicher Weise, wie dies für das Platinmetall weiter oben beschrieben wurde, bildet sich aus der metallischen Form dieser Zusätze und Halogenwasserstoff unter den Reaktionsbedingungen beispielsweise die Halogenidform solcher Zusätze. In bevorzugter Weise bestehen solche Zusätze aus einer Verbindung von Antimon, Wismut, Aluminium, Vanadium, Niob, Tantal oder einem Gemisch mehrerer von ihnen. Besonders bevorzugt liegen diese Zusätze als Halogenide von Antimon, Wismut, Aluminium, Vanadium, Niob, Tantal und insbesondere bevorzugt als Chloride von Antimon, Wismut, Aluminium, Vanadium, Niob und Tantal vor.

Die Menge des Zusatzes beträgt das 0,1-100fache, bevorzugt das 0,2-10fache der Menge an Platinmetall, gerechnet als Metall sowohl für den Zusatz als auch für das Platinmetall.

Die Herstellung eines erfindungsgemäß einzusetzenden Katalysators erfolgt nach Methoden, die dem Fachmann grundsätzlich bekannt sind. So kann der Träger mit einer Lösung einer der genannten Platinmetall-Verbindungen getränkt oder besprüht werden. In gleicher Weise wird mit dem oder den genannten Zusätzen verfahren. Für den Fall, daß das Platinmetall als Metall oder in Form des Carbonats, Oxids oder Hydroxids auf dem Träger fixiert werden soll und erst im Reaktor in der beschriebenen Weise mit Hilfe von Halogenwasserstoff unter den Reaktionsbedingungen zum Platinmetall-Halogenid aktiviert werden soll, kann die aufgetragene Platinmetall-Verbindung in einer dem Fachmann bekannten Weise durch ein geeignetes Reduktionsmittel zum Metall reduziert werden oder durch ein geeignetes Fällungsmittel in das Carbonat, Oxid oder Hydroxid umgewandelt werden.

Es wurde ferner beobachtet, daß es zur Erzielung gleichmäßig hoher Selektivitäten an Dialkylcarbonat vorteilhaft ist, Halogenwasserstoff an den Katalysator im Verlauf seiner Einsatzzeit heranzubringen. Hierbei wurde grundsätzlich gefunden, daß bei größerer Menge an Halogenwasserstoff die Ausbeute steigt. So kann die Konzentration an Halogenwasserstoff (z.B. HCl), die mit dem Einsatzmaterial an den Katalysator gebracht wird, beispielsweise bis zu 1000 ppm betragen. Es wurde jedoch weiter beobachtet, daß diese Menge an Halogenwasserstoff wesentlich kleiner sein kann. So ist es lediglich erforderlich, die Menge des mit den Reaktionsprodukten ausgetragenen, der aktivierten Form des Katalysators entstammenden Halogenwasserstoffs zu ersetzen. Diese Menge kann analytisch bestimmt werden. Sie bewegt sich im allgemeinen in einem Bereich von 1-2000 µg Halogenwasserstoff pro gebildetem g Dialkylcarbonat. Aus Gründen einer einfacheren Aufarbeitung kann es wünschenswert sein, wenig Halogenwasserstoff einzusetzen.

Halogenwasserstoff im Sinne der Erfindung ist Fluorwasserstoff, Chlorwasserstoff, Bromwasserstoff und Iodwasserstoff, bevorzugt Chlorwasserstoff und Bromwasserstoff, besonders bevorzugt Chlorwasserstoff.

Der Halogenwasserstoff kann als solcher gasförmig dem Reaktionsgemisch zudosiert werden. Er kann jedoch auch in gelöster Form in einem der im Reaktionsgemisch vorhandenen Stoffe eindosiert werden, so beispielsweise gelöst in dem dem Alkylnitrit zugrunde liegenden Alkohol.

Katalysatoren der beschriebenen Art haben eine hohe Standzeit (> 200 h). Über die bereits beschriebene mechanische Stabilität und Abriebfestigkeit hinaus behalten sie ihre Aktivität und Selektivität außerordentlich lange.

Die genannten Katalysatoren können mit 700-5 000 l Gemisch der gasförmigen Reaktionspartner pro l Katalysator und pro Stunde belastet werden (GHSV).

Das erfindungsgemäße Verfahren wird bei einer Temperatur von 50-150°C, bevorzugt 70-120°C, besonders bevorzugt 70-110°C, und einem Druck von 0,8-10 bar, bevorzugt 1-7 bar, besonders bevorzugt 1-5 bar, durchgeführt.

Die Herstellung der erfindungsgemäß einzusetzenden Alkylnitrite erfolgt nach bekannten Verfahren, beispielsweise aus dem zugehörigen Alkohol und salpetriger Säure, die etwa in situ aus einem Alkalinitrit und einer Mineralsäure, wie Schwefelsäure, gebildet wird. Das im Verlaufe des erfindungsgemäßen Verfahrens gebildete Stickstoffmonoxid NO kann kontinuierlich mit Sauerstoff und neuem Alkohol zu Alkylnitrit regeneriert werden (DE-OS 38 34 065) und gemeinsam mit nicht umgesetzten Reaktionspartnern recyclisiert werden.

Beispiele

Katalysatorherstellung und Definitionen

Vergleichsbeispiel 1

100 ml Aktivkohle-Granulat wurden in bekannter Weise mit einer Lösung von $PdCl_2$ und $CuCl_2$ in Wasser getränkt, das Produkt bei 80°C im Vakuum (20 Torr) getrocknet. Der fertige Katalysator enthielt 8 g Pd/l und 8 g Cu/l.

Die Raumzeitausbeute (Space Time Yield STY) in [g/l · h] und für Dimethylcarbonat in den Beispielen berechnet sich nach

$$\frac{m_{DMC}}{V_{Kat} \cdot t,}$$,

wobei $m_{DMC}$ die Menge gebildetes Dimethylcarbonat (DMC), $V_{Kat}$ das Katalysatorvolumen und t die Zeit bedeuten.

Die Selektivität S (%) wird berechnet nach

$$S = \frac{n_{DMC}}{n_{DMC} + 2 x n_{ODME} + n_{AME} + n_{FDA}} \times 100 \ [\%]$$

wobei

$n_{DMC}$ =  Stoffmenge Dimethylcarbonat
$n_{ODME}$ =  Stoffmenge Oxalsäuredimethylester
$n_{AME}$ =  Stoffmenge Ameisensäuremethylester
$n_{FDA}$ =  Stoffmenge Formaldehhyddimethylacetal

bedeuten.

Beispiel 1

100 ml $Al_2SiO_5$-Körper (Alumosilikat der ungefähren Zusammensetzung $Al_2O_3$ . $SiO_2$ mit einer spezifischen Oberfläche von 700 m²/g; PY 700 der Fa. Kali-Chemie) wurden mit einer wäßrigen $Li_2PdCl_4$-Lösung getrännt und das Produkt bei 80°C im Vakuum (29 Torr) getrocknet. Der Katalysator enthielt dann 8 g Pd/l.

Beispiel 2

In einem vertikal aufgestellten Glasrohr (Länge 50 cm, Durchmesser 4 cm) wurden zwischen eine Füllung aus Raschig-Ringen 20 ml des Katalysators aus Beispiel 1 gebracht.

Das Glasrohr wurde auf 90°C erhitzt und ein Gasgemisch aus 55 % $N_2$, 20 % MeONO, 20 % CO und 5 % MeOH geleitet Die Raumgeschwindigkeit betrug 800 l/l · h. Das dem Reaktor entströmende Gas wurde auf 5°C gekühlt und die erhaltene kondensierte Phase mittels Gaschromatographie untersucht. Die nicht kondensierten Produkte wurden mittels IR-Spektroskopie und Massenspektroskopie erfaßt.

Dimethylcarbonat wurde nach 2 h mit einer STY von 200 g/l · h und Selektivität = 99 % gebildet. Nach 10 Stunden betrug die STY 190 g/l · h und die Selektivität 99 %.

Vergleichsbeispiel 2

Der in Beispiel 2 beschriebene Versuch wurde wiederholt, wobei als Katalysator das in Vergleichsbeispiel 1 beschriebene Produkt eingesetzt wurde.

Dimethylcarbonat wurde nach 2 h mit einer STY von 80 g/l · h und einer Selektivität von 97 % gebildet. Nach 10 Stunden betrug die STY 60 g/l · h und die Selektivität 75 %.

**Patentansprüche**

**1.** Verfahren zur Herstellung von Dialkylcarbonaten der Formel

$$O=C(OR)_2,$$

worin

R    für geradkettiges oder verzweigtes $C_1$-$C_4$-Alkyl, bevorzugt für Methyl oder Ethyl, besonders bevorzugt für Methyl steht,

durch Umsetzung von Kohlenmonoxid (CO) mit Alkylnitriten der Formel

RONO,

worin

R    die angegebene Bedeutung hat,

in Anwesenheit oder Abwesenheit eines Inertgases sowie in Anwesenheit oder Abwesenheit des zugrundeliegenden Alkohols ROH sowie in Anwesenheit oder Abwesenheit von NO an einem Platinmetall-Träger-Katalysator bei erhöhter Temperatur in kontinuierlicher Gasphasenreaktion, dadurch gekennzeichnet, daß als Träger ein amorphes Alumosilikat mit einem Gehalt von 30 - 80 Atom-% Al, bezogen auf die Summe von Al und Si, eingesetzt werden, das Platinmetall in Form eines Halogenids oder einer Halogenid enthaltenden Komplexverbindung vorliegt, wobei das Platinmetallhalogenid oder der das Platinmetall enthaltende halogenhaltige Komplex in situ im Verfahrensreaktor aus dem Platinmetall oder einer halogenfreien Platinmetallverbindung mit Hilfe von Halogenwasserstoff unter den Reaktionsbedingungen gebildet werden kann, der Katalysator weiterhin mit oder ohne einen Zusatz aus einer Verbindung von Antimon, Wismut, Aluminium, Kupfer, Vanadium, Niob, Tantal, Zinn, Eisen, Kobalt, Nickel oder einem Gemisch mehrerer von ihnen ausgerüstet ist und bei einem Volumenverhältnis von Nitrit:CO = 0,1-10:1 und einer Temperatur von 50-150°C gearbeitet wird, wobei absatzweise oder kontinuierlich Halogenwasserstoff mindestens der Menge zugesetzt wird, die auch ohne Halogenwasserstoffzusatz mit dem Reaktionsgemisch aus dem Reaktor herausgetragen wird.

2.   Verfahren nach Anspruch 1, dadurch gekennzeichnet, daß bei 70-120°C, bevorzugt bei 70-110°C, gearbeitet wird.

3.   Verfahren nach Anspruch 1, dadurch gekennzeichnet, daß bei einem Volumenverhältnis Nitrit:CO = 0,2-4:1, bevorzugt 0,3-3:1, gearbeitet wird.

4.   Verfahren nach Anspruch 1, dadurch gekennzeichnet, daß als Platinmetall eines oder mehrere aus der Gruppe von Palladium, Platin, Iridium, Ruthenium und Rhodium, bevorzugt aus der Gruppe von Palladium, Ruthenium und Rhodium, besonders bevorzugt Palladium eingesetzt wird (werden).

5.   Verfahren nach Anspruch 1, dadurch gekennzeichnet, daß als Halogenid eines Platinmetall eines oder mehrere aus der Gruppe der einfachen oder komplexen Fluoride, Chloride, Bromide und Iodide, bevorzugt aus der Gruppe der Chloride und Bromide, besonders bevorzugt der Chloride eingesetzt wird (werden).

6.   Verfahren nach Anspruch 1, dadurch gekennzeichnet, daß der Katalysator mit einem Zusatz aus einer Verbindung von Antimon, Wismut, Aluminium, Vanadium, Niob, Tantal oder einem Gemisch mehrerer von ihnen, bevorzugt mit einem Zusatz aus einer Verbindung von Aluminium, ausgerüstet ist.

7.   Verfahren nach Anspruch 1, dadurch gekennzeichnet, daß in Anwesenheit eines Inertgases gearbeitet wird, wobei das Inertgas 20-80 Vol.-%, bevorzugt 30-70 Vol.-%, des gesamten Gasvolumens beträgt.

8.   Verfahren nach Anspruch 1, dadurch gekennzeichnet, daß bei einer Katalysatorbelastung von 700-5 000 l Gemisch der gasförmigen Reaktionspartner pro Stunde und pro l Katalysator gearbeitet wird.

9.   Verfahren nach Anspruch 1, dadurch gekennzeichnet, daß der Alumosilikat-Träger eine BET-Oberfläche von 100-1 000 $m^2$/g, bevorzugt 200-800 $m^2$/g hat.

10.  Verfahren nach Anspruch 1, dadurch gekennzeichnet, daß bei einem Druck von 0,8-10 bar, bevorzugt 1-7 bar, besonders bevorzugt 1-5 bar, gearbeitet wird.

**Claims**

1.   Process for the preparation of dialkyl carbonates of the formula

6

$$O=C(OR)_2,$$

in which
R represents straight-chain or branched $C_1$-$C_4$-alkyl, preferably methyl or ethyl, particularly preferably methyl, by reacting carbon monoxide (CO) with alkyl nitrites of the formula

$$RONO,$$

in which
R has the indicated meaning, in the presence or absence of an inert gas and in the presence or absence of the alcohol ROH on which the compounds are based, and in the presence or absence of NO, on a supported catalyst comprising platinum metal, at elevated temperature in a continuous gas phase reaction, characterised in that, as the support, an amorphous alumosilicate having a content of 30-80 atom% Al, based on the sum of Al and Si, is used, the platinum metal is present in the form of a halide or a halide-containing complex compound, where the platinum metal halide or the halogen-containing complex containing the platinum metal can be formed in situ in the process reactor from the platinum metal or a halogen-free platinum metal compound with the aid of hydrogen halide under the reaction conditions, the catalyst is further equipped or not equipped with an addition of a compound of antimony, bismuth, aluminium, copper, vanadium, niobium, tantalum, tin, iron, cobalt, nickel or a mixture of a plurality of these, and the process is carried out using a volume ratio of nitrite:CO = 0.1-10:1 and at a temperature from 50 to 150°C, hydrogen halide being added intermittently or continuously in at least that amount which is discharged, even if no hydrogen halide is added, from the reactor with the reaction mixture.

2.  Process according to Claim 1, characterised in that the process is carried out at from 70 to 120°C, preferably at from 70 to 110°C.

3.  Process according to Claim 1, characterised in that the process is carried out using a volume ratio of nitrite:CO=0.2-4:1, preferably 0.3-3:1.

4.  Process according to Claim 1, characterised in that the platinum metal used is (are) one or more from the group of palladium, platinum, iridium, ruthenium and rhodium, preferably from the group of palladium, ruthenium and rhodium, particularly preferably palladium.

5.  Process according to Claim 1, characterised in that the platinum metal halide used is (are) one or more from the group of the simple or complex fluorides, chlorides, bromides and iodides, preferably from the group of the chlorides and bromides, particularly preferably of the chlorides.

6.  Process according to Claim 1, characterised in that the catalyst is equipped with an addition of a compound of antimony, bismuth, aluminium, vanadium, niobium, tantalum or a mixture of a plurality thereof, preferably with an addition of an aluminium compound.

7.  Process according to Claim 1, characterised in that the process is carried out in the presence of an inert gas, the inert gas amounting to from 20 to 80% by volume, preferably from 30 to 70% by volume of the total gas volume.

8.  Process according to Claim 1, characterised in that the catalyst is charged with from 700 to 5,000 l of the mixture of gaseous reactants per hour and per l of catalyst.

9.  Process according to Claim 1, characterised in that the alumosilicate support has a BET area of 100-1000 $m^2$/g, preferably 200-800 $m^2$/g.

10. Process according to Claim 1, characterised in that the process is carried out at a pressure from 0.8 to 10 bar, preferably from 1 to 7 bar, particularly preferably from 1 to 5 bar.

**Revendications**

1.  Procédé de préparation de carbonate de dialkyle de formule :

$$O=C(OR)_2$$

dans laquelle

R représente un groupe alkyle en $C_1$-$C_4$, linéaire ou ramifié, de préférence un groupe méthyle ou éthyle, de façon particulièrement préférée le groupe méthyle, par réaction du monoxyde de carbone (CO) avec des nitrites d'alkyle de formule :

RONO

dans laquelle

R a le sens indiqué,

en présence ou en l'absence d'un gaz inerte, ainsi qu'en présence ou en l'absence de l'alcool ROH se trouvant à la base du composé ainsi qu'en présence ou en l'absence de NO sur un catalyseur à base d'un métal de la famille du platine sur un support, à une température élevée, dans une réaction continue en phase gazeuse, procédé caractérisé en ce qu'on utilise comme support un aluminosilicate amorphe ayant une teneur de 30 à 80 atomes % de Al, par rapport à la somme de Al et de Si, le métal de la famille du platine est présent sous forme d'un halogénure ou d'un composé complexe contenant de l'halogénure, l'halogénure de métal de la famille du platine ou le complexe halogéné contenant un métal de la famille du platine étant formé sur place ("in situ") dans les conditions de réaction dans le réacteur de mise en oeuvre du procédé, à partir du métal de la famille du platine ou d'un composé sans halogène de métal de la famille du platine, à l'aide d'un halogénure d'hydrogène ; le catalyseur comportant en outre ou ne comportant pas une addition d'un composé de l'antimoine, du bismuth, de l'aluminium, du cuivre, du vanadium, du niobium, du tantale, de l'étain, du fer, du cobalt, du nickel ou d'un mélange de plusieurs de ces composés, et le travail étant réalisé à un rapport volumétrique de nitrite : CO=0,1-10:1 et à une température de 50 à 150°C, avec addition discontinue ou en continu, de l'halogénure d'hydrogène introduit au moins en la quantité retirée avec le mélange réactionnel du réacteur, même sans addition d'halogénure d'hydrogène.

2. Procédé selon la revendication 1, caractérisé en ce qu'on travaille à 70-120°C, de préférence à 70-110°C.

3. Procédé selon la revendication 1, caractérisé en ce qu'on travaille selon un rapport volumétrique nitrite : CO = 0,2-4:1, de préférence 0,3-3:1.

4. Procédé selon la revendication 1, caractérisé en ce qu'on utilise, comme métal de la famille du platine, un ou plusieurs métaux choisis dans l'ensemble formé par le palladium, le platine, l'iridium, le ruthénium et le rhodium, de préférence dans l'ensemble formé par le palladium, le ruthénium et le rhodium, de façon particulièrement préférée le palladium.

5. Procédé selon la revendication 1, caractérisé en ce qu'on utilise, comme halogénure d'un métal de la famille du platine, un ou plusieurs halogénures choisis dans l'ensemble formé par des fluorures, des chlorures, des bromures et des iodures simples ou complexes, de préférence choisis dans l'ensemble formé par des chlorures et bromures, de façon particulièrement préférée les chlorures.

6. Procédé selon la revendication 1, caractérisé en ce que le catalyseur comporte une addition d'un composé de l'antimoine, du bismuth, de l'aluminium, du vanadium, du niobium, du tantale ou d'un mélange de plusieurs de ces composés, de préférence une addition d'un composé de l'aluminium.

7. Procédé selon la revendication 1, caractérisé en ce qu'on travaille en présence d'un gaz inerte, ce gaz inerte représentant 20 à 80 % en volume, de préférence 30 à 70 % en volume, du volume total des gaz.

8. Procédé selon la revendication 1, caractérisé en ce qu'on travaille avec une charge de catalyseur de 700 à 5 000 l du mélange des corps gazeux participant à la réaction, par heure et par litre de catalyseur.

9. Procédé selon la revendication 1, caractérisé en ce que le support en aluminosilicate possède une surface spécifique BET de 100 à 1 000 $m^2$/g, de préférence 200 à 800 $m^2$/g.

10. Procédé selon la revendication 1, caractérisé en ce qu'on travaille sous une pression de 0,8 à 10 bars, de préférence 1 à 7 bars, de façon particulièrement préférée 1 à 5 bars.